Europäisches Patentamt

European Patent Office

Office européen des brevets

(19)

(11) Veröffentlichungsnummer: **0 213 106**
**A2**

## (12) EUROPÄISCHE PATENTANMELDUNG

(21) Anmeldenummer: 86890097.8

(22) Anmeldetag: 07.04.86

(51) Int. Cl.⁴: **G01N 33/569** ,
//C12N5/00,C12P21/00

(30) Priorität: 13.08.85 AT 2362/85

(43) Veröffentlichungstag der Anmeldung:
04.03.87 Patentblatt 87/10

(84) Benannte Vertragsstaaten:
AT BE CH DE FR GB IT LI LU NL SE

(71) Anmelder: **Waldheim Pharmazeutika**
**Gesellschaft m.b.H.**
**Landeggerstrasse 7**
**A-2491 Neufeld/Leitha (Burgenland)(AT)**

(72) Erfinder: **Pöckl, Erich E., Dr.**
**Pöttschingerstrasse 23**
**A-7201 Neudörfl/L (Burgenland)(AT)**
Erfinder: **Haushofer, Alexander, Dr.**
**Eisslergasse 7**
**A-1130 Wien(AT)**

(74) Vertreter: **Beer, Otto, Dipl.-Ing. et al**
**Lindengasse 8**
**A-1071 Wien(AT)**

(54) **Verfahren zum Nachweis von Antikörpern und Verfahren zur Herstellung eines Mittels zum Nachweis von Antikörper.**

(57) Zum Nachweis von Antikörpern gegen Human-T-Lymphotropes Virus Typ III (HTLV-III) wird die Probe mit HTLV-III-Antigen, das auf einen Träger fixiert ist, kontaktiert, diejenigen Antikörper, die mit dem fixierten HTLV-III-Antigen keine Antikörper-Antigen Reaktion eingegangen sind, werden entfernt, der so behandelte Träger wird einem Antikörper, der mit einem fluoreszierenden Stoff konjugiert ist und der sich ausschließlich an den Antigen-Antikörper-Komplex anlagert, kontaktiert, worauf der nicht angelagerte Antikörper entfernt wird und die so behandelte Probe hinsichtlich des Auftretens von Fluoreszenz überprüft wird.

Zur Herstellung eines Mittels zum Nachweis von Antikörpern gewinnt man HTLV-III durch Züchten von virusproduzierenden T-Zell-Leukämie-Zellinien, transformiert mit den HTLV-III humane T-Zellinien, züchtet die transformierten humanen T-Zellinien zur Bildung von HTLV-III-Antigen und fixiert das HTLV-III-Antigen auf einem Träger.

## Verfahren zum Nachweis von Antikörpern und Verfahren zur Herstellung eines Mittels zum Nachweis von Antikörper

Die Erfindung betrifft ein Verfahren zum Nachweis von Antikörpern gegen Human-T-Lymphotropes Virus Typ III (HTLV-III) in Proben, insbesondere in Blutproben. Sie betrifft weiters ein Verfahren zur Herstellung eines Mittels zum Nachweis von Antikörpern, das insbesondere zur Ausführung des erfindungsgemäßen Verfahrens zum Nachweis von HTLV-III-Antikörpern geeignet ist.

Epidemiologische Daten weisen darauf hin, daß das Acquired Immunodeficiency Syndrome (AIDS) (Erworbenes Immunmangelsyndrom) durch ein infektiöses Agens verursacht wird, das durch Intimkontakt oder Exposition mit Blut oder bestimmten Blutprodukten horizontal übertragen wird. Bei Patienten mit AIDS, Prä-AIDS und bei gesunden Personen mit AIDS-Risiko wurden Retroviren isoliert, die zur Familie der humanen T-Lymphotropen-Viren - (HTLV) gehören und zusammengefaßt als HTLV-III bezeichnet werden.

Bisher wurden im wesentlichen zwei Reihenuntersuchungsverfahren (Screening Tests) zum Nachweis von HTLV-III-Antikörpern in Blutproben verwendet. Der von Wisdom, G.B.: Enzyme-Immunoassay Clin. Chem. 22/8, 1243-1255, (1976) und von McDougal, J.S. et al im Aufsatz Immunoassay for the Detection and Quantitation of Infectious Human Retrovirus, Lymphadenopathy-Associated Virus (LAV) in Journal of Immunological Methods 76, (1985) 171-183 beschriebene ELISA-Test (= Enzyme-Linked Immunosorbent-Assay) ist prinzipiell eine Doppelantikörpermethode. Antigen - (abgetötetes HTLV-III-Virus) wird an die Oberfläche von Polystyrolkörpern gebunden und mit den Blutproben inkubiert. Danach wird mit einem gekoppelten (Peroxidase) Maus-AntiHuman IgG inkubiert und die Gelbfärbung nach der Substratreaktion spektralphotometrisch (quantitativ) gemessen.

Der zweite Test ist der sogenannte "Western Blot" (vgl. Towbin, H. et al Electrophoretic transfer of proteins from polyacrylamide gels to nitrocellulose sheets: Procedure and some applications Proc. National Acad. Sci, USA 76. 4350-4354 - (1979) und Sarngadharan, M.G. et al Antibodies Reactive with Human T-Lymphotropic Retroviruses (HTLV-III) in the Serum of Patients with AIDS Science 224 , 506-508, (1984)), bei dem ein Lysat des HTLV-III auf einem SDS-Polyacrylamidgel - (SDS = Natriumdodecylsulfat) elektrophoretisch aufgetrennt wird. Danach werden die Proteinbanden des Gels elektrophoretisch auf ein Nitrozellulosefilter transferiert. Darauf wird diese mit Test-Sera inkubiert und nachher mit $^{125}$J-markiertem Ziegenanti-humanem IgG oder IgM inkubiert und nach der darauffolgenden Autoradiographie das Bandenmuster ausgewertet.

Für Forschungszwecke wurde im Labor manchmal die indirekte Cytospin Immunfluoreszenz (vgl. Goudsmit, J. et al Immunoglobulin Subclasses of Antibodies to Human T-Cell Leukemia/Lymphoma Virus I-Associated Antigens in Acquired Immune Deficiency Syndrome and Lymphadenopathy Syndrome J. Virol. 53. (1) 287-291, (1985)) angewendet. Dabei werden die HTLV-I produzierenden Zellen (C10/MJ2) mittels einer Cytospin-Zentrifuge direkt auf einen Objektträger zentrifugiert und nach dem Trocknen mit Testserum inkubiert. Dann wird mit FITC (Fluorescein-Isothiocyanat) gekoppeltem Kaninchenanti-humanem IgG oder anti-humanem IgM inkubiert und mittels Flow-Cytofluorimetrie gemessen.

Beim ELISA-Test treten -da er ungenau ist - viele sowohl falsch positive als auch fragliche Resultate auf, die durch den komplizierten und langwierigen "Western Blot" Test verifiziert werden müssen. Beide bekannten Verfahren eignen sich also nicht zur schnellen und sicheren Reihenuntersuchung (Screening) von Blutproben. Die indirekte Cytospin Immunfluoreszenz erreichte wegen ihrer Kompli ziertheit nie kommerzielle Bedeutung, zumal sie auch nur für den Nachweis des HTLV-I entwickelt wurde und nicht für HTLV-III, das aber das im erfindungsgemäßen Verfahren verwendete Virus ist.

Der Erfindung liegt die Aufgabe zugrunde, ein Verfahren der eingangs genannten Gattung zur Verfügung zu stellen, nach welchem sichere Reihenuntersuchungen von Proben, insbesondere von Blutproben, in kurzer Zeit möglich sind. Weiters stellt sich die Erfindung die Aufgabe, ein Verfahren zur Herstellung eines Mittels anzugeben, mit dem Antikörper nachgewiesen werden können.

Gemäß der Erfindung wird diese Aufgabe dadurch gelöst, daß man zum Nachweis von Antikörpern gegen HTLV-III folgende Verfahrensschritte anwendet:

a) Kontaktieren der Probe mit HTLV-III-Antigen, das von mit HTLV-III transformierten T-Zellen produziert und auf einen Träger fixiert ist,

b) Entfernen derjenigen Antikörper, die mit dem fixierten HTLV-IIIAntigen keine Antikörper-Antigen-Reaktion eingegangen sind,

c) Kontaktieren des so behandelten Trägers mit einem Antikörper, insbesondere einem Anti-Human Immunglobulin, der mit einem fluoreszierenden Stoff konjugiert ist und der sich ausschließlich an den Antigen-Antikörper-Komplex anlagert,

d) Entfernen des nicht angelagerten, mit einem fluoreszierenden Stoff konjugierten Antikörpers und

e) Prüfen der so behandelten Probe hinsichtlich des Auftretens von Fluoreszenz.

Das erfindungsgemäße Verfahren der indirekten Immunfluoreszenz (IFA) ist eine schnelle und vor allem sichere Diagnostik auf das Vorhandensein von HTLV-III-Antikörpern in Proben, im speziellen in Blutproben.

Besonders geeignet zur Durchführung von Antikörper-Reihenuntersuchungen, insbesondere zur Durchführung des erfindungsgemäßen Nachweisverfahrens ist ein Mittel, das gemäß der Erfindung wie folgt hergestellt wird, indem man

f) HTLV-III durch Züchten von virusproduzierenden T-Zell-Leukämie-Zellinien, z.B. CTCL-3, HUT-102, CR-CTC, EP/CTCL-5 oder AH/C-LAV-4 und nachfolgendem Isolieren des HTLV-III gewinnt,

g) mit den so gewonnenen HTLV-III humane T-Zellinien, die von Menschen mit akuter lymphatischer Leukämie isoliert wurden, wie z.B. TALL-I, PAL-EEP oder BL/ALL, transformiert und die transformierten humanen T-Zellinien zur Bildung von HTLV-III-Antigen züchtet und

h) das die transformierten humanen T-Zellinien enthaltende Medium zum Fixieren des HTLV-III-Antigens auf einem Träger, der vorzugsweise eine Mikrotiterplatte ist, mit einer Phosphatpufferlösung, z.B. 0,01 M PBS mit pH 7,3 wäscht, zentrifugiert, den Niederschlag, z.B. in 0,01 M PBS mit pH 7,3 resuspendiert und auf dem Träger z.B. mit Methanol/Azeton 50/50 und durch Abkühlen fixiert.

Einschließlich der Herstellung eines Mittels zur Durchführung des Verfahrens in Form einer Mikrotiterplatte können die erfindungsgemäßen Vorgangsweisen wie folgt an Hand eines Fallbeispieles beschrieben werden:

Das Virus HTLV-III wird in virusproduzierenden T-Zell-Leukämie-Zellinien, z.B. CTCL-3, HUT 102, CR-CTC, EP/CTCL-5, AH/C-LAV-4, vermehrt. Diese Zellinien werden in RPMI 1640 (ein Aminosäuren und Vitamine enthaltendes Nährmedium, das bei GIBCO erhältlich ist) mit 10 % hitze-inaktiviertem, totalem Kalbserum in Kulturflaschen nach den üblichen Zellkultur-Techniken gezüchtet.

Für diesen Test werden humane T-Zellinien verwendet, wie etwa TALL-I (6), PALL-EEP, BL/ALL-V, die von Patienten mit akuter lymphatischer Leukämie (= ALL) isoliert und dann gezüchtet wurden. Kulturmedium ist wieder RPMI 1640 mit 10 % hitze-inaktiviertem, totalem Kalbserum. $2 \times 10^7$ dieser Zellen werden mit 2 ml des zellfreien Überstandes der virusproduzierenden Zellen inokuliert und 2 -3 Wochen bei 37°C inkubiert, um die Zellen zu transformieren. Diese infizierten Zellen werden nun als Antigen für den Test verwendet. Sie werden mit 50 ml 0,001 M PBS (Phosphat-Pufferlösung), pH 7,3 (Hersteller: Flow-Laboratories) gewaschen und nach dem Abzentrifugieren bei 600 xg in einer Konzentration von $3 \times 10^9$ Zellen pro Liter in 0,01 M PBS, pH 7,3 resuspendiert. 50 $\mu$l der Zellsuspension ($3 \times 10^9$ Zellen pro Liter) werden in jedes Schälchen der Mikrotiterplatte gegeben und mit 0,25 ml Methanol/Azeton (50/50) versetzt. Danach wird 30 min bei -20°C fixiert und so aufbewahrt. Für den Test werden die Zellen mit 100 $\mu$l 0,01 M PBS, pH 7,3 für 10 min bei Zimmertemperatur rehydratisiert und dann im IFA-Test eingesetzt.

Die rehydratisierten Zellen werden mit 5 $\mu$l von 1 : 5 -Verdünnungen der aus den zu untersuchenden Blutproben gewonnenen Testsera überschichtet und für 30 min bei 37°C inkubiert. Danach wird mit PBS gewaschen und die Zellen mit 20 $\mu$l FITC-konjugiertem anti-humanem IgG - (Hersteller: Bio-Rad) bei 37°C für 30 min oder mit 20 $\mu$l FITC-konjugiertem anti-humanem IgM - (Hersteller: Bio-Rad) bei 37°C für 120 min inkubiert. Die Zellen werden dann mit PBS gewaschen und unter dem Fluoreszenz-Mikroskop ausgewertet. Das Auftreten einer Fluoreszenz zeigt an, daß die Probe HTLV-III-Antikörper enthält. Als negative Kontrolle dienen Zellen ohne Serum und als positive Kontrolle werden Zellen mit Serum eines verifizierten AIDS-Patienten, also mit Serum, das HTLV-III-Antikörper enthält, untersucht.

Die optische Auswertung der Tests wird wesentlich durch den Einsatz eines mikroprocessorgetesteten Scannersystems (Zusatz zum Mikroskop) erleichtert. Mit dieser Verbesserung eignet sich das erfindungsgemäße Untersuchungsverfahren besonders gut zur Massenuntersuchung.

Nach dem erfindungsgemäßen Verfahren und unter Verwendung von erfindungsgemäß hergestellten Mikrotiterplatten wurden Serumproben von 1353 freiwilligen Blutspendern aus sieben Blutspendezentralen und von 163 Blutern, 29 Personen, die mehrfach Bluttransfusionen erhielten und 53 männlichen Homosexuellen auf HTLV-III-Antikörper untersucht. Dabei zeigten alle 14 HTLV-III Positiv-Sera, die nach der Western Blotting Methode untersucht wurden, eine positive Reaktion auf HTLV-III IgG-Antikörper. Die Antikörper-Titer reichten von 160 bis zu 2560.

Anderseits zeigte das Serum der 1353 gesunden Erwachsenen eine negative Reaktion. Die Anti-ATLA Positiv-Sera (ATLA = Adult-T-cell Leukämie Virus I = HTLV-I) von vier Patienten mit ATL (= Adult-T-cell Leukämie) zeigten ebenfalls eine negative Reaktion. Nicht infizierte TALL-I Zellen in Zellausstrichen dienten zur Kontrolle für die Untersuchung der nicht virus-spezifischen Immunfluoreszenz. Insgesamt ist das Testsystem für HTLV-III hoch spezifisch.

Das Serum von 47 der 163 Bluter enthielt Anti-HTLV-III-IgG; das Serum von 10 dieser 163 Bluter enthielt auch Anti-ATLA Antikörper, drei hatten sowohl HTLV-III als auch Anti-ATLA Antikörper - (Tabelle I). Keine der Plasmaproben der 1353 freiwilligen Blutspender (Alter 16 -64 Jahre) aus den sieben Blutspendezentren enthielt Anti-LAV Antikörper (Tabelle II), jedoch enthielten 45 Anti-ATLA Antikörper. Die Serumproben von 53 männlichen Homosexuellen, ohne häufigen Partnerwechsel, wurden ebenfalls untersucht, keine enthielt jedoch HTLV-III Antikörper oder Anti-ATLA Antikörper. Von den 29 Patienten, die mehrfach Bluttransfusionen mit mehr als 50 Einheiten erhielten,besaß keiner HTLV-III Antikörper, aber 16 besaßen Anti-ATLA Antikörper.

### T a b e l l e  I

Aufscheinen von HTLV-III und Anti-ATLA Antikörper
im Serum von Blutern

--------------------------------------------------------------

|  | Anti-ATLA Antikörper | | |
|---|---|---|---|
|  | + | – | Total |
| HTLV-III Antikörper { + | 3 | 44 | 47 |
| – | 7 | 109 | 116 |
| t o t a l | 10 | 153 | 163 |

### T a b e l l e  II

Aufscheinen von HTLV-III und Anti-ATLA Antikörper
im Serum von freiwilligen Blutspendern

--------------------------------------------------------------

| getestete Spender | Spender mit Anti-ATLA-Antikörper *) | Spender mit HTLV-III Antikörper |
|---|---|---|
| 236 | 17 (7,2) | 0 |
| 204 | 14 (6,9) | 0 |
| 202 | 8 (4,0) | 0 |
| 200 | 3 (1,5) | 0 |
| 126 | 1 (0,8) | 0 |
| 186 | 0 (0) | 0 |
| 199 | 2 (1,0) | 0 |
| Total  1353 | 45 (3,3) | 0 |

(Prozentsatz in Klammer)

Die Isolierung der humanen T-Zellinien von Patienten mit akuter lymphatischer Leukämie - (TALL-I, PALL-EEP, BL/ALL-V) kann, wie von I. Miyoshi et al a.a.O. beschrieben, erfolgen.

Die virusproduzierenden Zellinien (CTCL-3, HUT 102, CR-CTC, EP/CTCL-5, AH/C-LAV-4) sind auf die gleiche Weise isoliert und dann auf ihre Infizierbarkeit mit HTLV-III mittels mehrerer Methoden (z.B. SDS-Polyacrylamid-Gelelektrophorese, direekte Immunfluoreszenz, reverse Transkriptase-Aktivität) überprüft worden (vgl. hiezu auch J.S. McDougal a.a.O.).

Nachstehend wird ein nicht beschränkendes Beispiel für das erfindungsgemäße Verfahren angegeben:

PALL-EEP-Zellen werden mit RPMI 1640 - (Hersteller: GIBCO) mit 10% hitzeinaktiviertem totalem Kälberserum nach den üblichen Zellkultur-Techniken gezüchtet. $2 \times 10^7$ dieser Zellen werden in einem sterilen Plastik-Zellkultur-Fläschchen unter sterilen Bedingungen mit 2 ml des zellfreien Überstandes der virusproduzierenden Zellen versetzt, zugeschraubt und in den Brutschrank bei 37°C gegeben. Nach einer Inkubationszeit von 15 Tagen sind die Zellen geeignet, um für den Test verwendet zu werden.

Der zellfreie Überstand der virusproduzierenden Zellen wurde, wie von J.S. McDougal et al a.a.O. beschrieben, gewonnen.

50 ml der infizierten PALL-EEP-Zellen werden bei 600 xg für 10 min zentrifugiert und der Überstand verworfen. Die Zellen werden in 50 ml 0,01 M PBS, pH 7,3 (Flow-Laboratories) aufgenommen und nochmals bei 600 xg für 10 min zentrifugiert. Dann werden die Zellen in 10 ml 0,01 M PBS, pH 7,3 suspendiert ($3 \times 10^9$ Zellen pro Liter) und in Aliquoten von 50 $\mu$l in Mikrotiterplatten aufgeteilt.

Dann wird jedes Schälchen mit 250 $\mu$l Methanol/Azeton (50/50) versetzt und für 30 min bei -20°C fixiert. Dann wird mit 100 $\mu$l 0,01 M PBS, pH 7,3 für 10 min bei Zimmertemperatur rehydratisiert. Das PBS wird dann abgesaugt und die Zellen werden mit 5 $\mu$l von 1 : 5-Verdünnungen (10 $\mu$l Serum auf 50 $\mu$l PBS) der Testsera überschichtet und für 30 min bei 37°C inkubiert. Das Serum wird mit 100 $\mu$l PBS weggewaschen und dann abgesaugt. Danach wird mit 20 $\mu$l FITC-konjugiertem Antihuman IgG (Bio-Rad) bei 37°C für 30 min oder mit 20 $\mu$l FITC-konjugiertem Anti-Human IgM (Bio-Rad) bei 37°C für 120 min inkubiert. Anschließend werden die Zellen mit 100 $\mu$l PBS gewaschen und die Proben unter dem Fluoreszenz-Mikroskop ausgewertet.

**Ansprüche**

1. Verfahren zum Nachweis von Antikörpern gegen Human-T-Lymphotropes Virus Typ III - (HTLV-III) in Proben, insbesondere in Blutproben, gekennzeichnet durch folgende Verfahrensschritte:

a) Kontaktieren der Probe mit HTLV-III-Antigen, das von mit HTLV-III transformierten T-Zellen produziert und auf einen Träger fixiert ist,

b) Entfernen derjenigen Antikörper, die mit dem fixierten HTLV-III-Antigen keine Antikörper-Antigen-Reaktion eingegangen sind,

c) Kontaktieren des so behandelten Trägers mit einem Antikörper, insbesondere einem Anti-Human Immunglobulin, der mit einem fluoreszierenden Stoff konjugiert ist und der sich ausschließlich an den Antigen-Antikörper-Komplex anlagert,

d) Entfernen des nicht angelagerten, mit einem fluoreszierenden Stoff konjugierten Antikörpers und

e) Prüfen der so behandelten Probe hinsichtlich des Auftretens von Fluoreszenz.

2. Verfahren nach Anspruch 1, dadurch gekennzeichnet, daß man in Stufe a) vorzugsweise verdünntes, aus der zu untersuchenden Blutprobe gewonnenes Serum verwendet und etwa 30 min lang bei 37°C inkubiert.

3. Verfahren nach Anspruch 1 oder 2, dadurch gekennzeichnet, daß man in den Stufen b) und/oder c) den Träger mit einer Pufferlösung, z.B. einem 0,01 M Phosphatpuffer mit pH 7,3 wäscht.

4. Verfahren nach einem der Ansprüche 1 bis 3, dadurch gekennzeichnet, daß man in Stufe c) ein Anti-Human IgG oder IgM, das z.B. von Ziegen oder Schafen stammt oder ein aus der Maus gewonnener monoklonaler Antikörper ist, das mit einem fluoreszierenden Stoff, z.B. mit Fluorescein-Isothiocyanat gekoppelt ist, verwendet.

5. Verfahren nach einem der Ansprüche 1 bis 4, dadurch gekennzeichnet, daß man Stufe e) unter Verwendung eines Mikroskopes durchführt.

6. Verfahren nach Anspruch 5, dadurch gekennzeichnet, daß man bei Verwendung einer Mikrotiterplatte als Träger für das HTLV-III Antigen ein Mikroskop verwendet, das mit einem Scanner ausgerüstet ist, der die Mikrotiterplatte scannt.

7. Verfahren zur Herstellung eines Mittels zum Nachweis von Antikörpern, insbesondere zur Durchführung des Verfahrens nach einem der Ansprüche 1 bis 6, dadurch gekennzeichnet, daß man

f) HTLV-III durch Züchten von virusproduzierenden T-Zell-Leukämie-Zellinien, z.B. CTCL-3, HUT-102, CR-CTC, EP/CTCL-5 oder AH/C-LAV-4 und nachfolgendem Isolieren des HTLV-III gewinnt,

g) mit den so gewonnenen HTLV-III humane T-Zellinien, die von Menschen mit akuter lymphatischer Leukämie isoliert wurden, wie z.B. TALL-I, PALL-EEP oder BL/ALL, transformiert und die transformierten humanen T-Zellinien zur Bildung von HTLV-III-Antigen züchtet und

h) das die transformierten humanen T-Zellinien enthaltende Medium zum Fixieren des HTLV-III-Antigens auf einem Träger, der vorzugsweise eine Mikrotiterplatte ist, mit einer Phosphatpufferlösung, z.B. 0,01 M PBS mit pH 7,3 wäscht, zentrifugiert, den Niederschlag z.B. in 0,01 M PBS, pH 7,3 resuspendiert und auf dem Träger, z.B. mit Methanol/Azeton 50/50 und durch Abkühlen fixiert.

8. Verfahren nach Anspruch 7, dadurch gekennzeichnet, daß man die virusproduzierenden T-Zell-Leukämie-Zellinien in einem Nährmedium mit 10 % hitze-inaktiviertem, totalem Kalbserum züchtet.

9. Verfahren nach Anspruch 7 oder 8, dadurch gekennzeichnet, daß man die humanen T-Zellinien zur Transformation mit dem zellfreien Nährmedium der virusproduzierenden T-Zell-Leukämie-Zellinien inokuliert und 1 -4, vorzugsweise 2 -3 Wochen lang bei 37°C inkubiert.

10. Verfahren nach einem der Ansprüche 7 bis 9, dadurch gekennzeichnet, daß man die humanen T-Zellinien in einem Nährmedium mit 10 % hitze-inaktiviertem, totalem Kalbserum züchtet.